# EUROPEAN PATENT APPLICATION

(11) **EP 4 600 249 A1**
(43) Date of publication of application: **13.08.2025**
(21) Application number: 24866546.5
(22) Date of filing: 24.10.2024
(51) Int. Cl.: C07F 7/00, C07C 215/68, C07C 217/84, C07D 213/73

(54) **NOVEL METAL-ORGANIC FRAMEWORK AND PREPARATION METHOD THEREOF**

(30) Priority: 28.12.2023 KR 20230194146
(71) Applicant: Mediark Inc., Seowon-gu Cheongju-si, Chungcheongbuk-do 28644 (KR)
(72) Inventor: KIM, Se Na, Sejong 30141 (KR); CHUN, Nea Young, Suwon-si, Gyeonggi-do 16510 (KR)
(74) Representative: BCKIP Part mbB
(86) International application number: PCT/KR2024/016238
(87) International publication number: WO 2025/143483

(57) **Abstract**

The present invention relates to a novel metal-organic framework (MOF) obtained by modifying the surface of a metal-organic framework, and a method for producing the same, wherein the novel surface-modified metal-organic framework has an increased specific surface area and a larger pore diameter compared to the metal-organic framework that has not been surface-modified. In addition, the surface-modified metal-organic framework produced by the method for producing a novel metal-organic framework according to the present invention has an excellent effect of binding to a nucleic acid and thus may be used as a nucleic acid delivery vehicle.

## Description

### Technical Field

The present invention relates to a novel metal-organic framework and a method for producing the same.

### Background Art

Organic-inorganic hybrid nanoporous materials, so-called "metal-organic frameworks (MOFs)", are generally referred to as "porous coordination polymers" or "porous organic-inorganic hybrid materials".

The metal-organic frameworks have recently been newly developed through the combination of molecular coordination bonding and material science. The metal-organic frameworks have recently been actively studied because they have a high surface area and molecular-sized or nano-sized pores, and thus are applicable to adsorbents, gas storage materials, sensors, membranes, functional thin films, drug delivery materials, catalysts, catalyst carriers, and the like, and they may be used to capture guest molecules smaller than the pore size or to separate molecules according to their sizes using the pores.

In addition, the metal-organic frameworks have the advantage of having nano-sized pores that provide a high surface area, and thus are mainly used for the purpose of adsorbing substances or delivering compositions by carrying the same in the pores.

For the above-described metal-organic frameworks, there is a need for the development of novel metal-organic frameworks with improved efficiency for various applications and methods for producing the same.

### [Prior Art Documents]

### [Patent Documents]

KR 10-2232255 B1

### DISCLOSURE

### Technical Problem

An object of the present invention is to provide a novel metal-organic framework (MOF) and a method for producing the same.

Another object of the present invention is to provide a novel metal-organic framework obtained by surface modification of a metal-organic framework, which has an increased specific surface area and a larger pore diameter compared to the metal-organic framework that has not been surface-modified.

Still another object of the present invention is to provide a method for producing a novel metal-organic framework, wherein the surface-modified metal-organic framework produced by the method has an excellent effect of binding to a nucleic acid and thus may be used as a nucleic acid delivery vehicle.

### Technical Solution

To achieve the above object, the present invention relates to a novel metal-organic framework surface-modified with a compound represented by Formula 1 below:

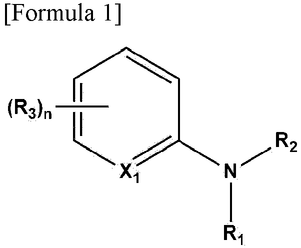

wherein
n is an integer ranging from 0 to 4,
X₁ is C(R₄) or N, and
R₁ to R₄ are the same as or different from one another and are each independently selected from the group consisting of hydrogen, a cyano group, a nitro group, a halogen group, a hydroxy group, a substituted or unsubstituted alkylthio group having 1 to 4 carbon atoms, a substituted or unsubstituted alkyl group having 1 to 30 carbon atoms, a substituted or unsubstituted cycloalkyl group having 3 to 20 carbon atoms, a substituted or unsubstituted alkenyl group having 2 to 30 carbon atoms, a substituted or unsubstituted alkynyl group having 2 to 24 carbon atoms, a substituted or unsubstituted aralkyl group having 7 to 30 carbon atoms, a substituted or unsubstituted aryl group having 6 to 30 carbon atoms, a substituted or unsubstituted heteroaryl group having 1 to 60 carbon atoms, a substituted or unsubstituted heteroarylalkyl group having 2 to 30 carbon atoms, a substituted or unsubstituted alkoxy group having 1 to 30 carbon atoms, a substituted or unsubstituted alkylamino group having 1 to 30 carbon atoms, a substituted or unsubstituted arylamino group having 6 to 30 carbon atoms, a substituted or unsubstituted aralkylamino group having 7 to 30 carbon atoms, a substituted or unsubstituted heteroarylamino group having 1 to 24 carbon atoms, a substituted or unsubstituted alkylsilyl group having 1 to 30 carbon atoms, a substituted or unsubstituted arylsilyl group having 6 to 30 carbon atoms, and a substituted or unsubstituted aryloxy group having 6 to 30 carbon atoms.

In addition, the compound represented by Formula 1 may be a compound represented by Formula 2 below:

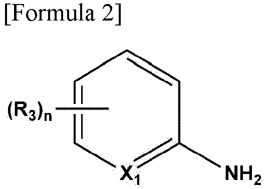

wherein
n, X₁ and R₃ are as defined in Formula 1 above.

In addition, the metal-organic framework may comprise a metal selected from the group consisting of Li, Na, Mg, Ca, Sr, Ba, Sc, Y, Ti, Zr, Hf, V, Nb, Ta, Cr, Mo, W, Mn, Re, Fe, Ru, Os, Co, Rh, Ir, Ni, Pd, Pt, Cu, Ag, Au, Zn, Cd, Hg, Al, Ga, In, Tl, Si, Ge, Sn, Pb, As, Sb and Bi, or a metal ion selected from the group consisting of Li⁺, Na⁺, Mg²⁺, Ca²⁺, Sr²⁺, Ba²⁺, Sc³⁺, Y³⁺, Ti⁴⁺, Zr⁴⁺, Hf⁴⁺, V⁴⁺, V³⁺, V²⁺, Nb³⁺, Ta³⁺ , Cr³⁺ , Mo³⁺, W³⁺, Mn³⁺ , Mn²⁺ , Re³⁺, Re²⁺ , Fe³⁺ , Fe ²⁺, Ru³⁺, Ru²⁺ ,Os³⁺, Os²⁺, Co³⁺, Co²⁺, Rh²⁺, Rh⁺, Ir³⁺, Ir²⁺, Ir⁺, Ni²⁺, Ni⁺, Pd²⁺, Pd⁺, Pt²⁺, Pt⁺, Cu²⁺, Cu⁺, Ag⁺, Au⁺, Zn²⁺, Cd²⁺, Hg²⁺, Al³⁺, Ga³⁺, In³⁺, Tl³⁺, Si⁴⁺, Si²⁺, Ge⁴⁺, Ge²⁺, Sn⁴⁺, Sn²⁺, Pb⁴⁺, Pb²⁺, As⁵⁺, As³⁺ , As⁺, Sb⁵⁺, Sb³⁺, Sb⁺, Bi⁵⁺, Bi³⁺ and Bi⁺.

In addition, the metal-organic framework may be selected from the group consisting of an aluminum-based metal-organic framework, an iron-based metal-organic framework, a zirconium-based metal-organic framework, and mixtures thereof.

In addition, the metal-organic framework may be selected from the group consisting of UIO-66, UIO-66-NH₂, UIO-67, UIO-67-NH₂, PCN-128, PCN-222, PCN-223, PCN-224, MOF-525, MOF-545, MOF-801, MOF-808, MOF-867, Al-MIL-53, Al-MIL-53-NH₂, Al-MIL-88, Al-MIL-88-NH₂, Al-MII,-100, Al-MIL-100-NH₂, Al-MIL-101, Al-MIL-101-NH₂, Al-MIL-125, Al-MIL-125-NH₂, Fe-MIL-53, Fe-MIL-53-NH₂, Fe-MIL-88, Fe-MIL-88-NH₂, Fe-MIL-100, Fe-MIL-100-NH₂, Fe-MIL-101, Fe-MIL-101-NH₂, Fe-MIL-125, and Fe-MIL-125-NH₂.

In addition, the metal-organic framework may have a specific surface area of 1,400 m²/g to 1,500 m²/g.

In addition, the metal-organic framework may have a pore diameter of 1 nm to 3 nm.

According to another embodiment of the present invention, the present invention relates to a method for producing a novel metal-organic framework, comprising steps of: preparing a metal-organic framework-containing solution by suspending a metal-organic framework in a first organic solvent; preparing a surface-modifying solution by dissolving a compound represented by Formula 1 below in a second organic solvent; and mixing and stirring the metal-organic framework-containing solution and the surface-modifying solution:

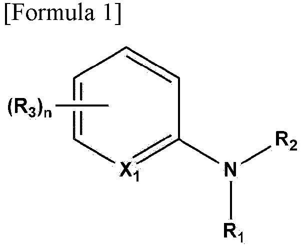

wherein
n is an integer ranging from 0 to 4,
X₁ is C(R₄) or N, and
R₁ to R₄ are the same as or different from one another and are each independently selected from the group consisting of hydrogen, a cyano group, a nitro group, a halogen group, a hydroxy group, a substituted or unsubstituted alkylthio group having 1 to 4 carbon atoms, a substituted or unsubstituted alkyl group having 1 to 30 carbon atoms, a substituted or unsubstituted cycloalkyl group having 3 to 20 carbon atoms, a substituted or unsubstituted alkenyl group having 2 to 30 carbon atoms, a substituted or unsubstituted alkynyl group having 2 to 24 carbon atoms, a substituted or unsubstituted aralkyl group having 7 to 30 carbon atoms, a substituted or unsubstituted aryl group having 6 to 30 carbon atoms, a substituted or unsubstituted heteroaryl group having 1 to 60 carbon atoms, a substituted or unsubstituted heteroarylalkyl group having 2 to 30 carbon atoms, a substituted or unsubstituted alkoxy group having 1 to 30 carbon atoms, a substituted or unsubstituted alkylamino group having 1 to 30 carbon atoms, a substituted or unsubstituted arylamino group having 6 to 30 carbon atoms, a substituted or unsubstituted aralkylamino group having 7 to 30 carbon atoms, a substituted or unsubstituted heteroarylamino group having 1 to 24 carbon atoms, a substituted or unsubstituted alkylsilyl group having 1 to 30 carbon atoms, a substituted or unsubstituted arylsilyl group having 6 to 30 carbon atoms, and a substituted or unsubstituted aryloxy group having 6 to 30 carbon atoms.

In addition, the step of stirring may be performed by stirring at 80°C to 120°C for 4 to 8 hours to surface-modify the metal-organic framework.

In addition, the step of stirring may be followed by washing and drying steps, thereby producing a surface-modified metal-organic framework.

In addition, the washing step may comprise steps of: subjecting a reaction product resulting from the step of stirring to a first centrifugation at 5,000 rpm to 15,000 rpm for 1 to 10 minutes; suspending a precipitate, separated by the first centrifugation, in a third organic solvent, and then subjecting the suspension to a first washing by repeating a second centrifugation at 5,000 rpm to 15,000 rpm for 1 to 10 minutes 1 to 5 times; and suspending a precipitate, separated by the first washing, in a fourth organic solvent, and then subjecting the suspension to a second washing by repeating a second centrifugation at 5,000 rpm to 15,000 rpm for 1 to 10 minutes 1 to 5 times.

In addition, the drying step may be performed by drying lower-layer particles, separated by the second washing, in an oven at 60°C to 100°C for 2 to 6 hours.

In the present invention, the "nucleic acid" is RNA, DNA, short interfering RNA (siRNA), messenger RNA (mRNA), an aptamer, an antisense oligodeoxynucleotide (ODN), an antisense RNA, a ribozyme, or DNAzyme.

In the present invention, "hydrogen" is hydrogen, protium, deuterium, or tritium, without being particularly limited thereto.

In the present invention, the "halogen group" is fluorine, chlorine, bromine, or iodine.

In the present invention, the "alkyl" means a monovalent substituent derived from a straight or branched chain saturated hydrocarbon having 1 to 40 carbon atoms. Examples thereof include, but are not limited to, methyl, ethyl, propyl, isobutyl, sec-butyl, pentyl, iso-amyl, hexyl, and the like.

In the present invention, the "alkenyl" means a monovalent substituent derived from a straight or branched chain unsaturated hydrocarbon having 2 to 40 carbon atoms and having at least one carbon-carbon double bond. Examples thereof include, but are not limited to, vinyl, allyl, isopropenyl, 2-butenyl, and the like.

In the present invention, the "alkynyl" means a monovalent substituent derived from a straight or branched chain unsaturated hydrocarbon having 2 to 40 carbon atoms and having at least one carbon-carbon triple bond. Examples thereof include, but are not limited to, ethynyl, 2-propynyl, and the like.

In the present invention, the "alkylthio" means the above-described alkyl group bonded through a sulfur linkage (-S-).

In the present invention, the "aryl" means a monovalent substituent derived from an aromatic hydrocarbon having 6 to 60 carbon atoms and having a single ring or a combination of two or more rings. In addition, it may also include a form in which two or more rings are simply attached (pendant) or fused, and specific examples thereof include, but are not limited to, a naphthyl group, an anthracenyl group, a phenanthryl group, a triphenyl group, a pyrenyl group, a phenalenyl group, a perylenyl group, a chrysenyl group, fluorenyl group, etc.. The fluorenyl group may be substituted, and adjacent groups may be bonded to each other to form a ring.

In the present invention, the "heteroaryl" means a monovalent substituent derived from a monoheterocyclic or polyheterocyclic aromatic hydrocarbon having 6 to 30 carbon atoms. Here, at least one carbon in the ring, preferably 1 to 3 carbons, is/are substituted with a heteroatom such as N, O, S or Se. In addition, the heteroaryl may include a form in which two or more rings are simply attached (pendant) or fused. Furthermore, it may include a form fused with an aryl group. Examples of such heteroaryls include, but are not limited to, 6-membered monocyclic rings such as pyridyl, pyrazinyl, pyrimidinyl, pyridazinyl, and triazinyl; polycyclic rings such as phenoxathienyl, indolizinyl, indolyl, purinyl, quinolyl, benzothiazole, and carbazolyl; and 2-furanyl, N-imidazolyl, 2-isoxazolyl, 2-pyridinyl, 2-pyrimidinyl, etc.

In the present invention, the "aryloxy" means a monovalent substituent represented by RO-, wherein R is an aryl having 6 to 60 carbon atoms. Examples of the aryloxy include, but are not limited to, phenyloxy, naphthyloxy, diphenyloxy, and the like.

In the present invention, the "alkoxy" may be linear, branched or cyclic. The number of carbon atoms of the alkoxy is not particularly limited, but is preferably 1 to 20 carbon atoms. Specifically, the alkoxy may be methoxy, ethoxy, n-propoxy, isopropoxy, i-propyloxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy, or the like, without being limited thereto.

In the present invention, the "aralkyl" means an aryl-alkyl group, wherein the aryl and the alkyl are as defined above. Preferred aralkyls include lower alkyl groups. Non-limiting examples of suitable aralkyl groups include benzyl, 2-phenethyl, and naphthalenylmethyl. Bonding to the parent moiety is via the alkyl.

In the present invention, the "arylamino group" means an amine substituted with an aryl group having 6 to 30 carbon atoms.

In the present invention, the "alkylamino group" means an amine substituted with an alkyl group having 1 to 30 carbon atoms.

In the present invention, the "aralkylamino group" means an amine substituted with an aryl-alkyl group having 6 to 30 carbon atoms.

In the present invention, the "heteroarylamino group" means an amine group substituted with an aryl group having 6 to 30 carbon atoms and a heterocyclic group.

In the present invention, the "heteroaralkyl group" means an aryl-alkyl group substituted with a heterocyclic group.

In the present invention, the "cycloalkyl" means a monovalent substituent derived from a monocyclic or polycyclic non-aromatic hydrocarbon having 3 to 40 carbon atoms. Examples of the cycloalkyl include, but are not limited to, cyclopropyl, cyclobutyl, cyclopentyl, cyclohexyl, norbornyl, adamantine, and the like.

In the present invention, the "heterocycloalkyl" means a monovalent substituent derived from a non-aromatic hydrocarbon having 3 to 40 carbon atoms, wherein at least one carbon in the ring, preferably 1 to 3 carbons, is/are substituted with a heteroatom such as N, O, S or Se. Examples of the heterocycloalkyl include, but are not limited to, morpholine, piperazine, and the like.

In the present invention, the "alkylsilyl" means silyl substituted with alkyl having 1 to 40 carbon atoms, and "arylsilyl" means silyl substituted with aryl having 6 to 60 carbon atoms.

In the present invention, the "fused ring" means a fused aliphatic ring, a fused aromatic ring, a fused heteroaliphatic ring, a fused heteroaromatic ring, or a combination thereof.

In the present invention, "adjacent groups are bonded to each other to form a ring" means that adjacent groups are bonded to each other to form a substituted or unsubstituted aliphatic hydrocarbon ring; a substituted or unsubstituted aromatic hydrocarbon ring; a substituted or unsubstituted aliphatic heterocyclic ring; a substituted or unsubstituted aromatic heterocyclic ring; or a fused ring thereof.

In the present invention, examples of the "aromatic hydrocarbon ring" include, but are not limited to, a phenyl group, a naphthyl group, an anthracenyl group, etc.

In the present invention, the "aliphatic heterocyclic ring" means an aliphatic ring containing at least one heteroatom.

In the present invention, "aromatic heterocyclic ring" means an aromatic ring containing at least one heteroatom.

In the present invention, "substituted" means that a hydrogen atom bonded to a carbon atom of a compound is replaced with another substituent, and the position of substitution is not limited as long as it is a position where a hydrogen atom is substituted, that is, a position where a hydrogen atom may be substituted with a substituent. When two or more hydrogen atoms are substituted, the two or more substituents may be the same as or different from each other. The substituent may be at least one substituent selected from the group consisting of hydrogen, a cyano group, a nitro group, a halogen group, a hydroxy group, an alkyl group having 1 to 30 carbon atoms, an alkenyl group having 2 to 30 carbon atoms, an alkynyl group having 2 to 24 carbon atoms, a heteroalkyl group having 2 to 30 carbon atoms, an aralkyl group having 6 to 30 carbon atoms, an aryl group having 5 to 30 carbon atoms, a heteroaryl group having 2 to 30 carbon atoms, a heteroarylalkyl group having 3 to 30 carbon atoms, an alkoxy group having 1 to 30 carbon atoms, an alkylamino group having 1 to 30 carbon atoms, an arylamino group having 6 to 30 carbon atoms, an aralkylamino group having 6 to 30 carbon atoms, a heteroarylamino group having 2 to 24 carbon atoms, a substituted or unsubstituted alkylsilyl group having 1 to 30 carbon atoms, a substituted or unsubstituted arylsilyl group having 6 to 30 carbon atoms, and a substituted or unsubstituted aryloxy group having 6 to 30 carbon atoms, without being limited to these examples.

### Advantageous Effects

The present invention provides a novel metal-organic framework obtained by modifying the surface of a metal-organic framework, wherein the novel metal-organic framework has an increased specific surface area and a larger average pore diameter compared to the metal-organic framework that has not been surface-modified.

The present invention also provides a method for producing a novel metal-organic framework, wherein the surface-modified metal-organic framework produced by the method has an excellent effect of binding to a nucleic acid and thus may be used as a nucleic acid delivery vehicle.

### Brief Description of Drawings

FIG. 1 is a schematic view of a metal-organic framework according to one embodiment of the present invention.
FIG. 2 shows the results of scanning electron microscope observation of a metal-organic framework according to one embodiment of the present invention.
FIG. 3 shows the results of X-ray diffraction analysis of a metal-organic framework according to one embodiment of the present invention.
FIG. 4 shows the results of BET analysis by N₂ adsorption/desorption of a metal-organic framework according to one embodiment of the present invention.
FIG. 5 shows the results of FT-IR measurement of a metal-organic framework according to one embodiment of the present invention.
FIG. 6 shows the results of 13C-NMR measurement of a metal-organic framework according to one embodiment of the present invention.
FIG. 7 is a schematic view of a metal-organic framework according to one embodiment of the present invention.
FIG. 8 shows the results of scanning electron microscope observation of a metal-organic framework according to one embodiment of the present invention.
FIG. 9 shows the results of X-ray diffraction analysis of a metal-organic framework according to one embodiment of the present invention.
FIG. 10 shows the results of BET analysis by N₂ adsorption/desorption of a metal-organic framework according to one embodiment of the present invention.
FIG. 11 shows the results of FT-IR measurement of a metal-organic framework according to one embodiment of the present invention.
FIG. 12 shows the results of 13C-NMR measurement of a metal-organic framework according to one embodiment of the present invention.
FIG. 13 is a schematic view of a metal-organic framework according to one embodiment of the present invention.
FIG. 14 shows the results of scanning electron microscope observation of a metal-organic framework according to one embodiment of the present invention.
FIG. 15 shows the results of X-ray diffraction analysis of a metal-organic framework according to one embodiment of the present invention.
FIG. 16 shows the results of BET analysis by N₂ adsorption/desorption of a metal-organic framework according to one embodiment of the present invention.
FIG. 17 shows the results of FT-IR measurement of a metal-organic framework according to one embodiment of the present invention.
FIG. 18 shows the results of 13C-NMR measurement of a metal-organic framework according to one embodiment of the present invention.
FIG. 19 shows the results of electrophoresis of an mRNA-MOF complex according to one embodiment of the present invention.
FIG. 20 shows the results of electrophoresis of an mRNA-MOF complex according to one embodiment of the present invention.

### Best Mode

The present invention relates to a novel metal-organic framework surface-modified with a compound represented by Formula 1 below:

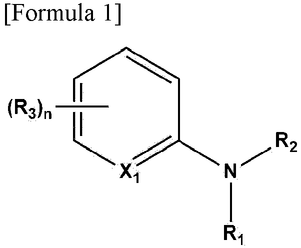

wherein
n is an integer ranging from 0 to 4,
X₁ is C(R₄) or N, and
R₁ to R₄ are the same as or different from one another and are each independently selected from the group consisting of hydrogen, a cyano group, a nitro group, a halogen group, a hydroxy group, a substituted or unsubstituted alkylthio group having 1 to 4 carbon atoms, a substituted or unsubstituted alkyl group having 1 to 30 carbon atoms, a substituted or unsubstituted cycloalkyl group having 3 to 20 carbon atoms, a substituted or unsubstituted alkenyl group having 2 to 30 carbon atoms, a substituted or unsubstituted alkynyl group having 2 to 24 carbon atoms, a substituted or unsubstituted aralkyl group having 7 to 30 carbon atoms, a substituted or unsubstituted aryl group having 6 to 30 carbon atoms, a substituted or unsubstituted heteroaryl group having 1 to 60 carbon atoms, a substituted or unsubstituted heteroarylalkyl group having 2 to 30 carbon atoms, a substituted or unsubstituted alkoxy group having 1 to 30 carbon atoms, a substituted or unsubstituted alkylamino group having 1 to 30 carbon atoms, a substituted or unsubstituted arylamino group having 6 to 30 carbon atoms, a substituted or unsubstituted aralkylamino group having 7 to 30 carbon atoms, a substituted or unsubstituted heteroarylamino group having 1 to 24 carbon atoms, a substituted or unsubstituted alkylsilyl group having 1 to 30 carbon atoms, a substituted or unsubstituted arylsilyl group having 6 to 30 carbon atoms, and a substituted or unsubstituted aryloxy group having 6 to 30 carbon atoms.

### Mode for Invention

Hereinafter, embodiments of the present invention will be described in detail so that those skilled in the art to which the present invention pertains can easily carry out the present invention. However, the present invention may be embodied in various different forms and is not limited to the embodiments described herein.

In the present invention, the metal-organic framework (MOF) refers to a porous material in which metal clusters and organic linkers (or organic bridging ligands) are connected by coordination bonds to form a three-dimensional structure. Various MOFs may be created depending on the choice of the metal ion and the organic ligand.

The MOF is characterized by a porous property in which pores exist in the structure, and the pore size, porosity, three-dimensional structure, surface area, etc. of the MOF may be designed in various ways depending on the types of and the method of bonding between the metal ion and organic ligand forming the MOF.

Due to this porosity, the MOFs not only have a very large surface area, but also have an open pore structure, allowing the movement of a large amount of molecules or solvents therethrough compared to other porous materials known to date. In addition, when the MOFs are used as catalysts or gas storage materials, they may advantageously maximize efficiency due to their numerous active sites. In addition, the MOFs are not easily deformed at high temperatures and have a rigid skeleton, so they have excellent chemical and thermal stability.

The present invention relates to a novel MOF, which is not a conventionally known MOF, but is a MOF obtained by modifying the surface of the MOF. As described below, the MOF surface-modified with a novel compound is characterized by having a larger surface area and a larger pore diameter than the conventional MOF. Unlike the conventional MOF, the novel MOF has been surface-modified with a novel compound, and therefore, as described below, it more easily binds to a nucleic acid and may also be used as a more effective *in vivo* nucleic acid delivery vehicle.

That is, although it is known that the conventional MOF may be used as a drug delivery vehicle due to its large surface area and numerous pores, but the present invention may facilitate the binding of the MOF to a nucleic acid by modifying the surface of the MOF, thereby increasing the efficiency of the MOF as a nucleic acid delivery vehicle.

In the case of conventional LNPs for delivering nucleic acids *in vivo,* temperature conditions during storage are important due to particle instability, but the novel MOF of the present invention as described above may be used as a more stable nucleic acid delivery vehicle.

Specifically, the novel MOF of the present invention may be one surface-modified with a compound represented by Formula 1 below:

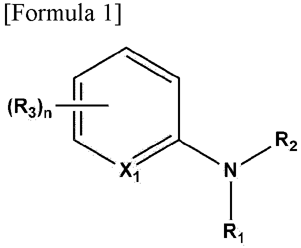

wherein
n is an integer ranging from 0 to 4,
X₁ is C(R₄) or N, and
R₁ to R₄ are the same as or different from one another and are each independently selected from the group consisting of hydrogen, a cyano group, a nitro group, a halogen group, a hydroxy group, a substituted or unsubstituted alkylthio group having 1 to 4 carbon atoms, a substituted or unsubstituted alkyl group having 1 to 30 carbon atoms, a substituted or unsubstituted cycloalkyl group having 3 to 20 carbon atoms, a substituted or unsubstituted alkenyl group having 2 to 30 carbon atoms, a substituted or unsubstituted alkynyl group having 2 to 24 carbon atoms, a substituted or unsubstituted aralkyl group having 7 to 30 carbon atoms, a substituted or unsubstituted aryl group having 6 to 30 carbon atoms, a substituted or unsubstituted heteroaryl group having 1 to 60 carbon atoms, a substituted or unsubstituted heteroarylalkyl group having 2 to 30 carbon atoms, a substituted or unsubstituted alkoxy group having 1 to 30 carbon atoms, a substituted or unsubstituted alkylamino group having 1 to 30 carbon atoms, a substituted or unsubstituted arylamino group having 6 to 30 carbon atoms, a substituted or unsubstituted aralkylamino group having 7 to 30 carbon atoms, a substituted or unsubstituted heteroarylamino group having 1 to 24 carbon atoms, a substituted or unsubstituted alkylsilyl group having 1 to 30 carbon atoms, a substituted or unsubstituted arylsilyl group having 6 to 30 carbon atoms, and a substituted or unsubstituted aryloxy group having 6 to 30 carbon atoms.

The compound represented by Formula 1 may be a compound represented by Formula 2 below:

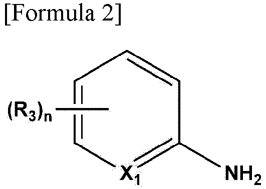

wherein
n, X₁ and R₃ are as defined in Formula 1 above.

The compound represented by Formula 2 may be a compound represented by any one of Formulas 3 to 5 below:

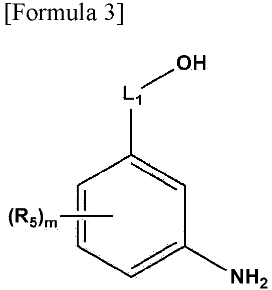

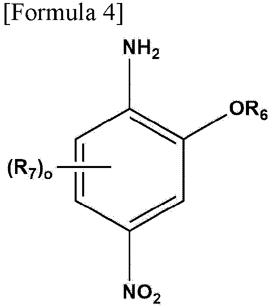

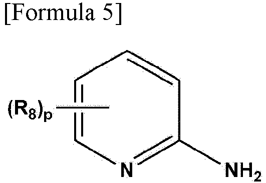

wherein
m and p are the same as or different from each other and are each independently an integer ranging from 0 to 4;
o is an integer ranging from 0 to 3;
L₁ is selected from the group consisting of a single bond, a substituted or unsubstituted alkylene group having 1 to 10 carbon atoms, a substituted or unsubstituted cycloalkylene group having 3 to 10 carbon atoms, a substituted or unsubstituted alkenylene group having 2 to 10 carbon atoms, and a substituted or unsubstituted cycloalkenylene group having 3 to 10 carbon atoms; and
R₅ to R₈ are the same as or different from one another and are each independently selected from the group consisting of a hydrogen, a cyano group, a nitro group, a halogen group, a hydroxy group, a substituted or unsubstituted alkylthio group having 1 to 4 carbon atoms, a substituted or unsubstituted alkyl group having 1 to 30 carbon atoms, a substituted or unsubstituted cycloalkyl group having 3 to 20 carbon atoms, a substituted or unsubstituted alkenyl group having 2 to 30 carbon atoms, a substituted or unsubstituted alkynyl group having 2 to 24 carbon atoms, a substituted or unsubstituted aralkyl group having 7 to 30 carbon atoms, a substituted or unsubstituted aryl group having 6 to 30 carbon atoms, a substituted or unsubstituted heteroaryl group having 1 to 60 carbon atoms, a substituted or unsubstituted heteroarylalkyl group having 2 to 30 carbon atoms, a substituted or unsubstituted alkoxy group having 1 to 30 carbon atoms, a substituted or unsubstituted alkylamino group having 1 to 30 carbon atoms, a substituted or unsubstituted arylamino group having 6 to 30 carbon atoms, a substituted or unsubstituted aralkylamino group having 7 to 30 carbon atoms, a substituted or unsubstituted heteroarylamino group having 1 to 24 carbon atoms, a substituted or unsubstituted alkylsilyl group having 1 to 30 carbon atoms, a substituted or unsubstituted arylsilyl group having 6 to 30 carbon atoms, and a substituted or unsubstituted aryloxy group having 6 to 30 carbon atoms.

The compound represented by Formula 1 may be selected from the group consisting of compounds represented by Formulas below:

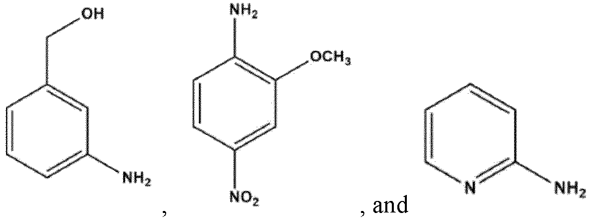

N of the -NH₂ group commonly contained in the above compounds contains an unshared electron pair and may form a coordination bond with the MOF by the unshared electron pair, so that each of the compounds may bind to the surface of the MOF.

As described above, according to the present invention, it is possible to surface-modify the MOF through bonding by the unshared electron pair of each of the compounds.

The MOF may comprise a metal selected from the group consisting of Li, Na, Mg, Ca, Sr, Ba, Sc, Y, Ti, Zr, Hf, V, Nb, Ta, Cr, Mo, W, Mn, Re, Fe, Ru, Os, Co, Rh, Ir, Ni, Pd, Pt, Cu, Ag, Au, Zn, Cd, Hg, Al, Ga, In, Tl, Si, Ge, Sn, Pb, As, Sb and Bi, or a metal ion selected from the group consisting of Li⁺, Na⁺, Mg²⁺, Ca²⁺, Sr²⁺, Ba²⁺, Sc³⁺, Y³⁺, Ti⁴⁺, Zr⁴⁺, Hf⁴⁺, V⁴⁺, V³⁺, V²⁺, Nb³⁺, Ta³⁺, Cr³⁺, Mo³⁺, W³⁺, Mn³⁺, Mn²⁺ , Re³⁺, Re²⁺, Fe³⁺, Fe²⁺, Ru³⁺, Ru²⁺, Os³⁺, Os²⁺, Co³⁺, Co²⁺, Rh²⁺, Rh⁺, Ir³⁺, Ir ²⁺, Ir⁺, Ni²⁺, Ni⁺, Pd²⁺, Pd⁺, Pt²⁺, Pt⁺, Cu²⁺, Cu⁺, Ag⁺, Au⁺, Zn²⁺, Cd²⁺, Hg²⁺, Al³⁺, Ga³⁺, In³⁺, T1³⁺, Si⁴⁺, Si²⁺, Ge⁴⁺, Ge²⁺, Sn⁴⁺, Sn²⁺, Pb⁴⁺, Pb²⁺ , As⁵⁺, As³⁺, As⁺, Sb⁵⁺, Sb³⁺, Sb⁺, Bi⁵⁺, Bi³⁺ and Bi⁺.

The metal-organic framework may be selected from the group consisting of an aluminum-based MOF, an iron-based MOF, a zirconium-based MOF, and mixtures thereof.

The MOF may be selected from the group consisting of UIO-66, UIO-66-NH₂, UIO-67, UIO-67-NH₂, PCN-128, PCN-222, PCN-223, PCN-224, MOF-525, MOF-545, MOF-801, MOF-808, MOF-867, Al-MIL-53, Al-MIL-53-NH₂, Al-MIL-88, Al-MIL-88-NH₂, Al-MIL-100, Al-MIL-100-NH₂, Al-MIL-101, Al-MIL-101-NH₂, Al-MIL-125, Al-MIL-125-NH₂, Fe-MIL-53, Fe-MIL-53-NH₂, Fe-MIL-88, Fe-MIL-88-NH₂, Fe-MIL-100, Fe-MIL-100-NH₂, Fe-MIL-101, Fe-MIL-101-NH₂, Fe-MIL-125, and Fe-MIL-125-NH₂, with UIO-66 being preferred, but the metal-organic framework is not limited to the above examples, and any MOF capable of forming a coordination bond with the compound represented by Formula 1 may be used without limitation.

In addition, the MOF may have a specific surface area of 1,400 m²/g to 1,500 m²/g and a pore diameter of 1 nm to 3 nm. The MOF of the present invention is characterized by having a larger specific surface area and pore diameter than a non-surface-modified MOF due to its surface modification.

The MOF of the present invention may be used as a nucleic acid delivery vehicle as described above. Here, the nucleic acid is specifically mRNA, but is not limited to the above example, and any nucleic acid that may be bound to the surface-modified MOF of the present invention may be used without limitation. The MOF of the present invention may easily bind to a nucleic acid and may be stably released after being injected *in vivo,* and thus it may be used as an excellent nucleic acid delivery vehicle.

A method for producing a novel metal-organic framework according to another embodiment of the present invention may comprise steps of: preparing a metal-organic framework-containing solution by suspending a metal-organic framework in a first organic solvent; preparing a surface-modifying solution by dissolving a compound represented by Formula 1 below in a second organic solvent; and mixing and stirring the metal-organic framework-containing solution and the surface-modifying solution:

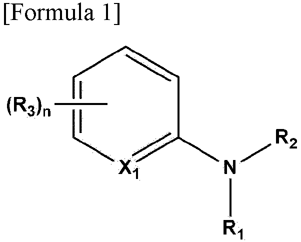

wherein
n is an integer ranging from 0 to 4,
X₁ is C(R₄) or N, and
R₁ to R₄ are the same as or different from one another and are each independently selected from the group consisting of hydrogen, a cyano group, a nitro group, a halogen group, a hydroxy group, a substituted or unsubstituted alkylthio group having 1 to 4 carbon atoms, a substituted or unsubstituted alkyl group having 1 to 30 carbon atoms, a substituted or unsubstituted cycloalkyl group having 3 to 20 carbon atoms, a substituted or unsubstituted alkenyl group having 2 to 30 carbon atoms, a substituted or unsubstituted alkynyl group having 2 to 24 carbon atoms, a substituted or unsubstituted aralkyl group having 7 to 30 carbon atoms, a substituted or unsubstituted aryl group having 6 to 30 carbon atoms, a substituted or unsubstituted heteroaryl group having 1 to 60 carbon atoms, a substituted or unsubstituted heteroarylalkyl group having 2 to 30 carbon atoms, a substituted or unsubstituted alkoxy group having 1 to 30 carbon atoms, a substituted or unsubstituted alkylamino group having 1 to 30 carbon atoms, a substituted or unsubstituted arylamino group having 6 to 30 carbon atoms, a substituted or unsubstituted aralkylamino group having 7 to 30 carbon atoms, a substituted or unsubstituted heteroarylamino group having 1 to 24 carbon atoms, a substituted or unsubstituted alkylsilyl group having 1 to 30 carbon atoms, a substituted or unsubstituted arylsilyl group having 6 to 30 carbon atoms, and a substituted or unsubstituted aryloxy group having 6 to 30 carbon atoms.

The MOF is as described above and may be produced using a conventional MOF production method, and there is no limitation on the method for producing the MOF.

Specifically, a metal-organic framework-containing solution may be prepared by suspending the MOF in a first organic solvent. Here, the first organic solvent is toluene, but is not limited to toluene, and any organic solvent in which the MOF may be suspended to prepare the metal-organic framework-containing solution may be used without limitation.

In a next step, a surface-modifying solution may be prepared by dissolving the compound represented by Formula 1 in a second organic solvent. The compound represented by Formula 1 is as described above. The compound represented by Formula 1 may be a purchased compound or a compound synthesized by a conventional synthesis method.

The second organic solvent is toluene, but is not limited to toluene, and any organic solvent in which the compound represented by Formula 1 may be dissolved to prepare as the surface-modifying solution may be used without limitation.

Thereafter, the metal-organic framework-containing solution and the surface-modifying solution may be mixed together and stirred. The stirring step may be performed by stirring at 80°C to 120°C for 4 to 8 hours so that the compound represented by Formula 1 may be bound to the surface of the metal-organic framework through a coordination bond.

The above stirring conditions may be stirring at 80°C to 120°C for 4 to 8 hours, or stirring at 90°C to 110°C for 5 to 7 hours, or stirring at 100°C for 6 hours. Under these stirring conditions, the compound represented by Formula 1 may be coordinated to the surface of the MOF.

The stirring step may be followed by washing and drying steps, thereby producing a surface-modified metal-organic framework.

More specifically, the washing step may comprise steps of: subjecting a reaction product resulting from the stirring step to a first centrifugation at 5,000 rpm to 15,000 rpm for 1 to 10 minutes; suspending a precipitate, separated by the first centrifugation, in a third organic solvent, and then subjecting the suspension to a first washing by repeating a second centrifugation at 5,000 rpm to 15,000 rpm for 1 to 10 minutes 1 to 5 times; and suspending a precipitate, separated by the first washing, in a fourth organic solvent, and then subjecting the suspension to a second washing by repeating a second centrifugation at 5,000 rpm to 15,000 rpm for 1 to 10 minutes 1 to 5 times.

The third organic solvent may be toluene, and the fourth organic solvent may be ethanol, but the third and fourth organic solvents are not limited to the above examples, and any organic solvents capable of washing the synthesized surface-modified MOF may be used without limitation.

In addition, the drying step may be performed by drying lower-layer particles, separated by the second washing, in an oven at 60°C to 100°C for 2 to 6 hours.

### Production Example

### Production of Novel MOF

Benzoic acid (11.2 g) and benzene-1,4-dicarboxylic acid (BDC) were added to 250 ml of N,N-dimethylformamide (DMF), followed by stirring. 2.12 g of zirconium (IV) chloride (ZrCl₄) was added to the solution which was then stirred. Thereafter, the mixture was allowed to react in an oven at 120°C for 24 hours. The reaction product was collected and centrifuged at 10,000 rpm for 5 minutes, and the supernatant was removed. A washing process of resuspending the centrifuged precipitate in 30 ml of DMF and centrifuging the suspension at 10,000 rpm for 5 minutes was repeated three times. The DMF was removed, and a washing process of resuspending the precipitate in 30 ml of ethanol and centrifuging the suspension at 10,000 rpm for 5 minutes was repeated 3 times. The ethanol was removed, and the lower-layer particles were dried in an oven at 80°C for 4 hours to obtain powder (UIO-66).

200 mg of the powder obtained above was taken and suspended in 10 ml of toluene, thus preparing a toluene suspension.

3-aminobenzyl alcohol (ABA), 2-methoxy-4-nitroaniline (MN), and 2-aminopyridine (APD) were each taken in amounts of 0.6 mmol and then dissolved in 40 ml of toluene, thereby preparing toluene solutions.

The toluene suspension and each of the toluene solution were placed in a round-bottom flask, the flask was connected to a reflux condenser, and the contents in the flask were stirred at 100°C for 6 hours, thus producing a reaction product.

The reaction product was collected and centrifuged at 10,000 rpm for 5 minutes, and the supernatant was removed. The centrifuged precipitate was resuspended in 30 ml of toluene, and a washing process of centrifuging the suspension at 10,000 rpm for 5 minutes was repeated three times.

The same washing process was repeated three times using ethanol instead of toluene.

In the final washing process, the supernatant ethanol was removed, and the lower-layer particles were dried in an oven at 80°C for 4 hours to obtain the final product powder.

The product obtained by surface-modification with ABA was referred to as UIO66ABA, the product obtained by surface modification with MN was referred to as UIO66MN, and the product obtained by surface modification with APD was referred to as UIO66APD.

### Experimental Example 1

### Analysis of Synthesis Results

For measurement using a scanning electron microscope (SEM), 2 mg to 3 mg of a powder sample were placed in an Eppendorf tube and 100 µl of ethanol was added thereto, followed by bath sonication. Thereafter, 10 µl was dropped on a silicon wafer and dried in an oven at 80°C. The silicon wafer with the dried sample was attached to a SEM mount using carbon tape, and the sample was imaged using a SEM instrument (Zeiss, Model ULTRA PLUS).

Next, for X-ray diffraction (XRD) measurement, a powder sample was carefully placed on the XRD sample holder, and the sample was compressed by pressing the same with an appropriate force using slide glass. Thereafter, all powder scattered around the sample holder was carefully removed, and the sample holder was transferred to an X-ray diffractometer (Bruker, model D2 Phaser) and set, and then measurements were performed in the range of 2°C to 30°C.

Next, for N₂ adsorption/desorption isotherm, BET surface area and porosity measurements, about 40 mg of a completely dried sample was prepared and carefully placed in a glass sampler using a glass funnel, and all powder accumulated around the glass tube outside the lower sample container at the bottom was removed. Thereafter, an upper filter cap was attached and degassing was performed under vacuum by heat treatment at 120°C for 12 hours in a pretreatment instrument. The pretreated sample was weighed using a microbalance, and the correct weight of the sample was calculated by subtracting the premeasured weight of the empty cell. The glass sampler was placed on an N₂ adsorption-desorption instrument (MICROTRAC, model BELSORP MINI X), and measurements were performed.

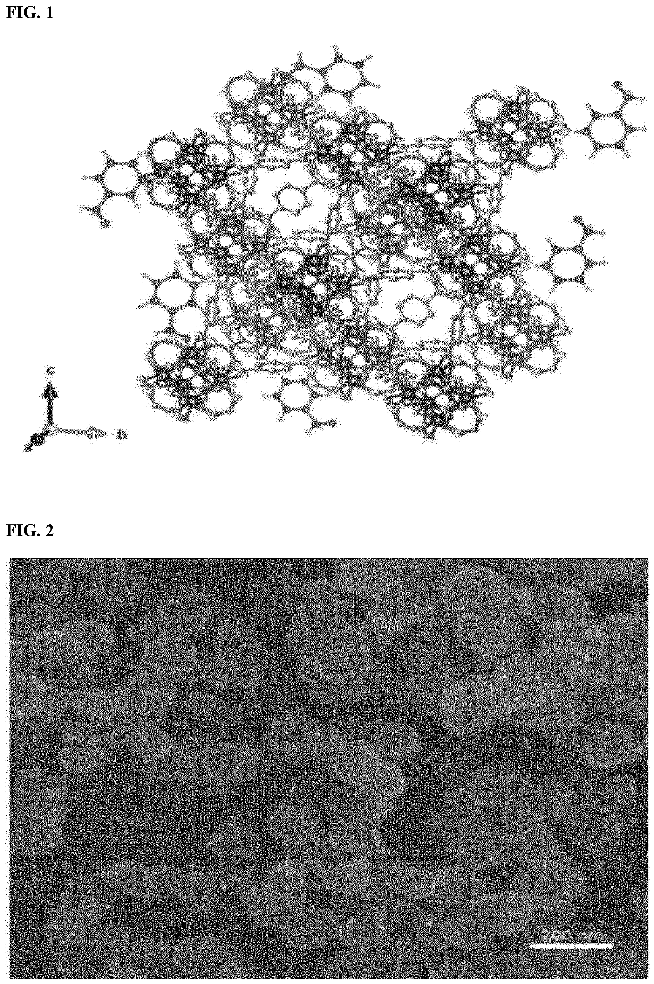

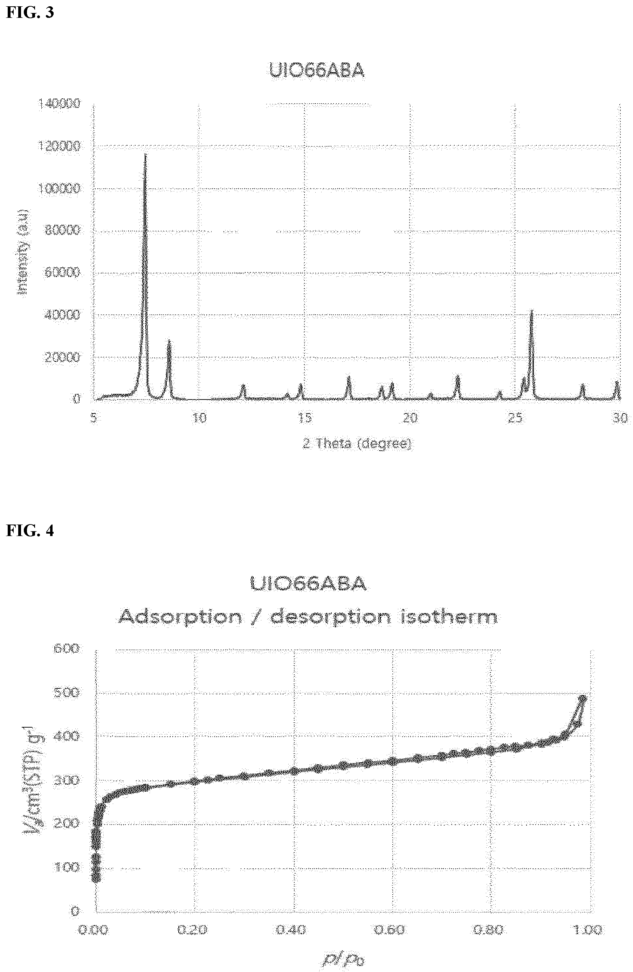

FIG. 1 schematically shows the particle structure of UIO66ABA. FIG. 2 shows the results of scanning electron microscope observation of UIO66ABA. In addition, the results of X-ray diffraction analysis are shown in FIG. 3 and confirm that the produced particles were synthesized with the crystal structure of UIO66ABA. In addition, the results of BET (Brunauer-Emmett-Teller) analysis through N₂ adsorption/desorption showed that the specific surface area of UIO66ABA particles was 1,481 m²/g and the pore diameter thereof was 2.110 nm (FIG. 4).

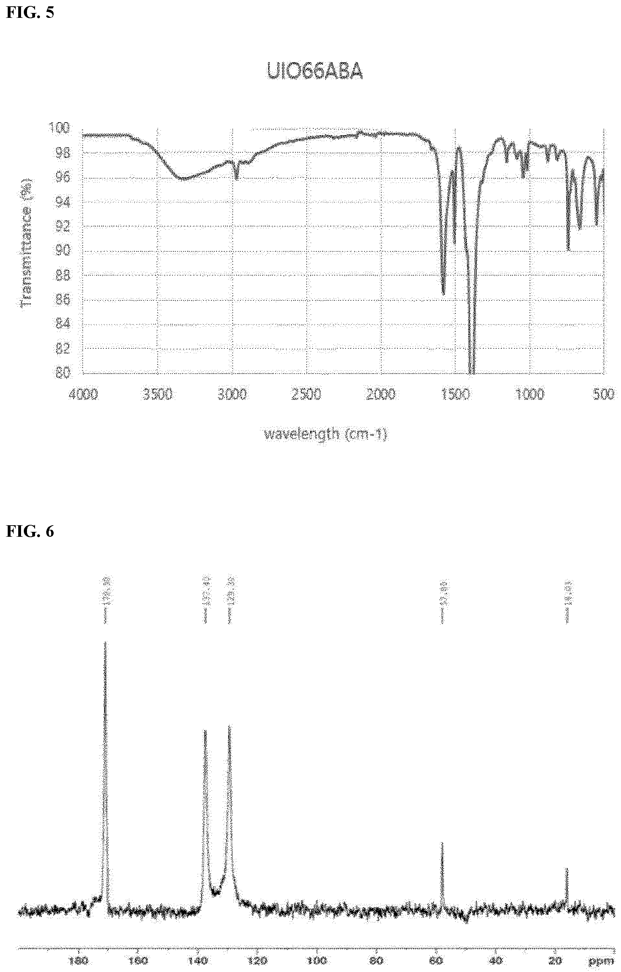

The results of FT-IR measurement of UIO66ABA are shown in FIG. 5. FIG. 6 shows the results of 13C-NMR measurement of the synthesized UIO66ABA particles.

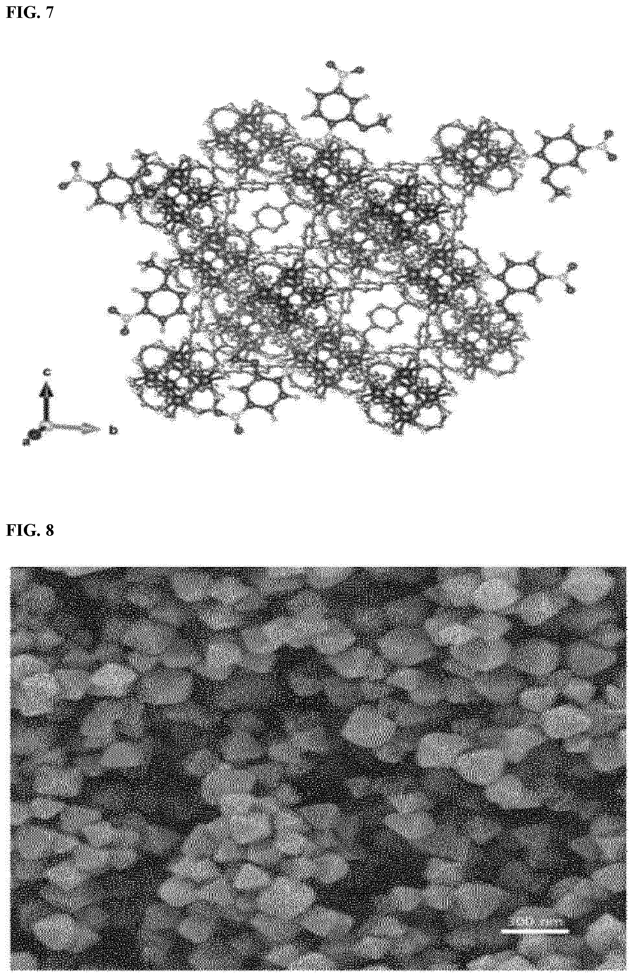

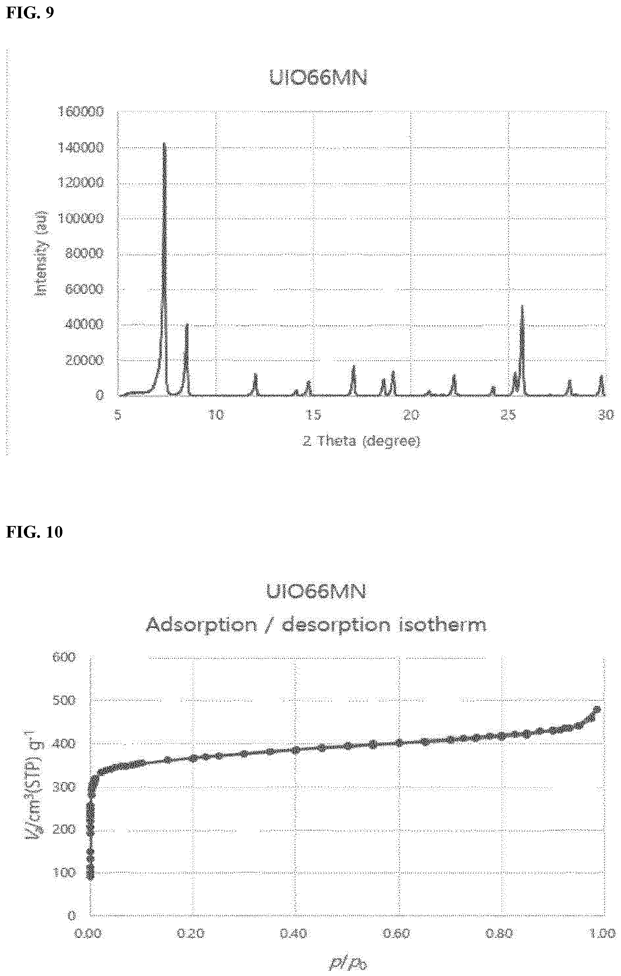

FIG. 7 schematically shows the particle structure of UIO66MN. FIG. 8 shows the results of scanning electron microscope observation of UIO66MN. In addition, the results of X-ray diffraction analysis are shown in FIG. 9 and confirm that the produced particles were synthesized with the crystal structure of UIO66MN. In addition, the results of BET (Brunauer-Emmett-Teller) through N₂ adsorption/desorption showed that the specific surface area of UIO66MN particles was 1,449 m²/g and the pore diameter thereof was 2.044 nm (FIG. 10).

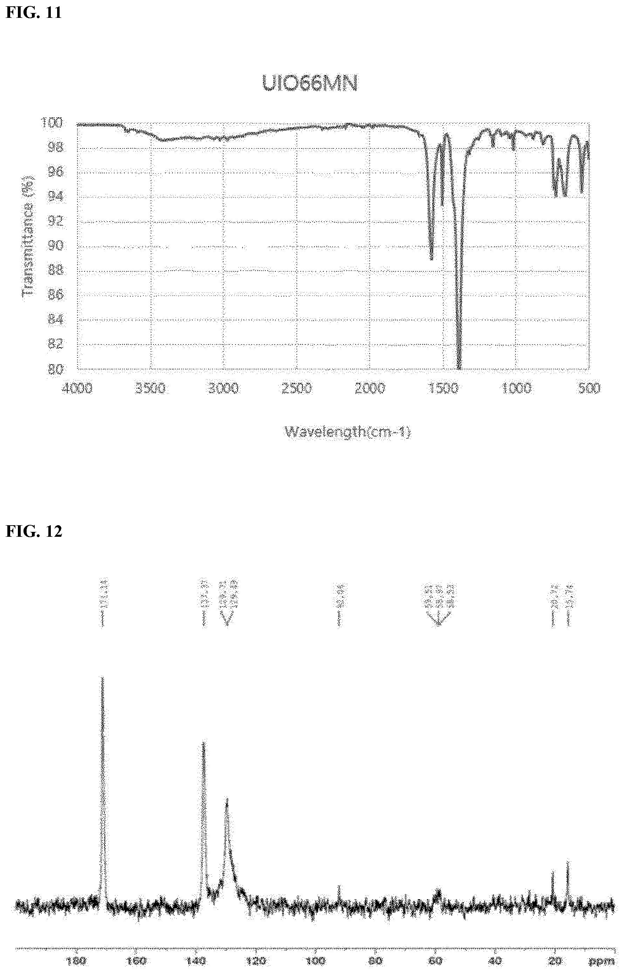

The results of FT-IR measurement of UIO66MN are shown in FIG. 11. FIG. 12 shows the results of 13C-NMR measurement of the synthesized UIO66MN particles.

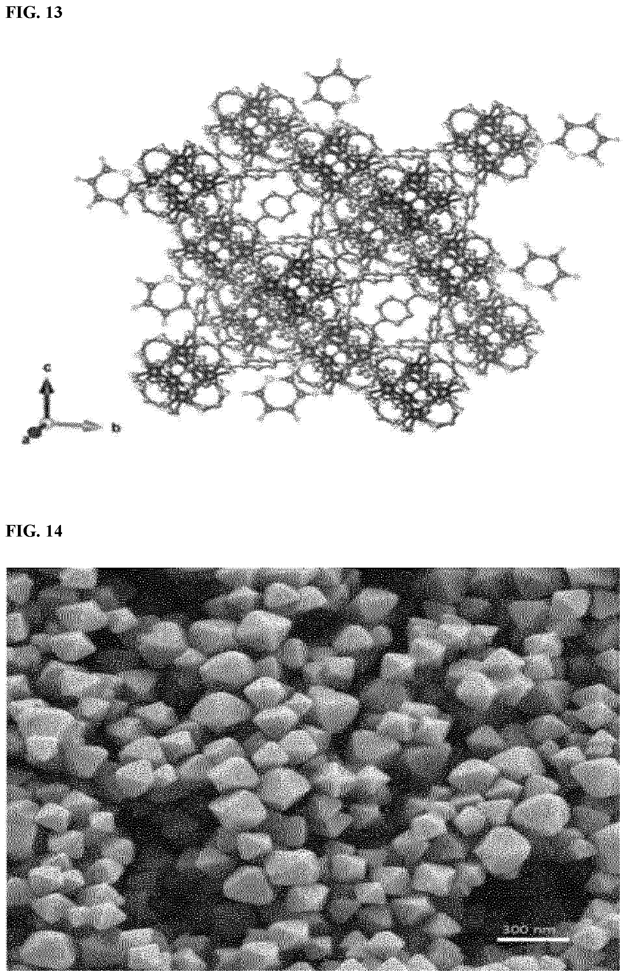

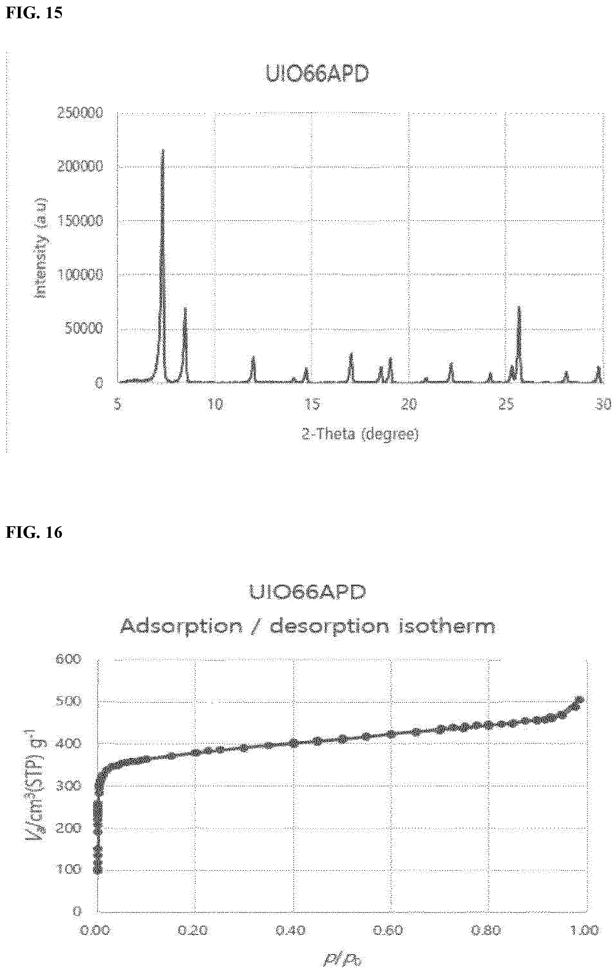

FIG. 13 schematically shows the particle structure of UIO66APD. FIG. 14 shows the results of scanning electron microscope observation of UIO66APD. In addition, the results of X-ray diffraction analysis are shown in FIG. 15 and confirm that the produced particles were synthesized with the crystal structure of UIO66APD. In addition, the results of BET (Brunauer-Emmett-Teller) analysis through N₂ adsorption/desorption showed that the specific surface area of UIO66APD particles was 1,481 m²/g and the pore diameter thereof was 2.110 nm (FIG. 16).

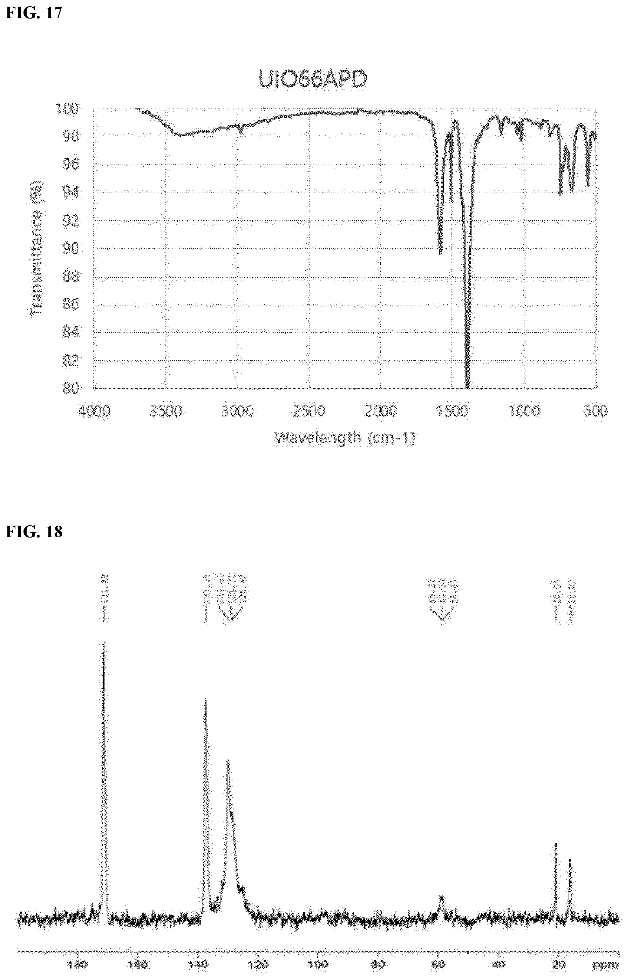

The results of FT-IR measurement of UIO66APD are shown in FIG. 17. FIG. 18 shows the results of 13C-NMR measurement of the synthesized UIO66APD particles.

The specific surface area of UIO-66 is generally known to be 1,180 m²/g to 1,240 m²/g. It was confirmed that the specific surface area of the UIO66ABA particles of the present invention was 1,481 m²/g, the specific surface area of the UIO66MN particles was 1,449 m²/g, and the specific surface area of the UIO66APD particles was 1,481 m²/g, indicating that the specific surface areas were significantly different from that of UIO-66 that has not been surface-modified.

In addition, the pore diameter of UIO-66 is 0.6 nm, and as described above, the pore diameter of the UIO66ABA particles was 2.110 nm, the pore diameter of the UIO66MN particles was 2.044 nm, and the pore diameter of the UIO66APD particles was 2.110 nm, indicating that the pore diameters were significantly different from that of UIO-66 that has not been surface-modified.

### Experimental Example 2

### Experiment for Formation of m-RNA-MOF Complex and Confirmation of Formation of m-RNA-MOF Complex

1.0 g of agarose was added to 100 ml of 1X TAE buffer and dissolved at high temperature, thereby preparing a 1% agarose gel. 7 µl of Dyne StainingSTAR (EtBr replacement) was added to and mixed thoroughly with the agarose gel solution, and the resulting solution was poured and solidified in an electrophoresis gel forming mold.

The solidified agarose gel was placed in an electrophoresis device, and 1X TAE buffer was added to sufficiently submerge the gel.

UIO66MN and UIO66APD were each suspended in DW at a concentration of 10 mg/ml, and then diluted to concentrations of 10 mg/ml, 5 mg/ml, 2.5 mg/ml, 1.25 mg/ml, 0.625 mg/ml, and 0.312 mg/ml.

200 ng/µl mRNA solution was prepared by adding 80 µl of 10 mM NaOAC to 20 ul of mRNA (1 µg/µl). 1 µl of the mRNA solution and 10 µl of each of the UIO66MN and UIO66APD solutions were mixed together and incubated at room temperature for 10 minutes, thereby preparing mRNA-MOF complexes (mRNA-UIO66MN complex and mRNA-UIO66APD complex).

An mRNA control was prepared by mixing 1 µl of the mRNA solution and 10 µl of DI.

5 µl of DNA ladder (50 bp) and 1 µl of DNA loading buffer were mixed together, and then 5 µl of the mixture was loaded onto the agarose gel. 5 µl of each of the mRNA control and the mRNA-MOF complexes (mRNA-UIO66MN complex and mRNA-UIO66APD complex) was mixed with 1 µl of DNA loading buffer, and then 5 µl of the mixture was loaded onto the agarose gel.

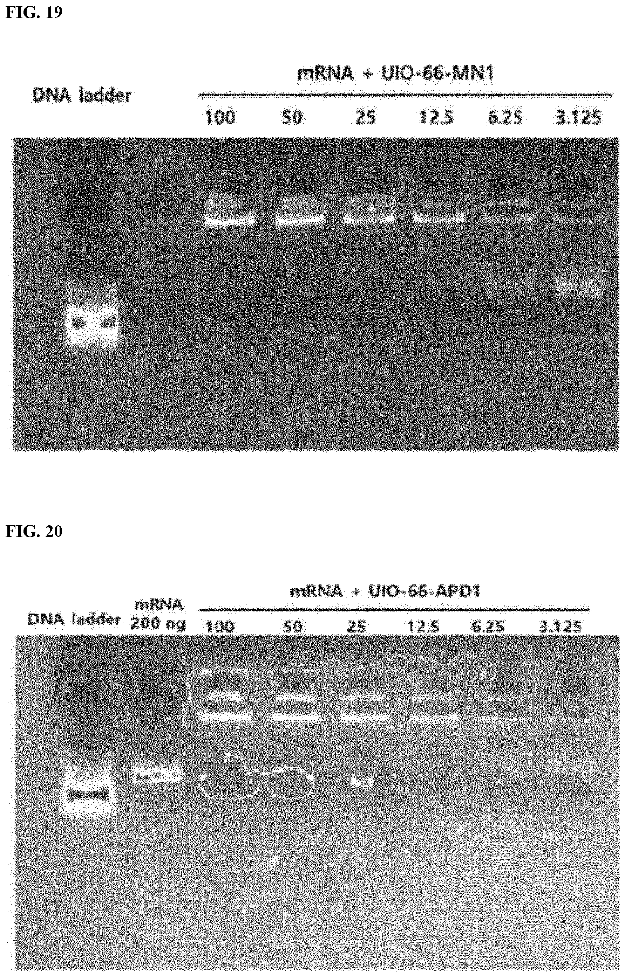

FIGS. 19 and 20 show the results of performing electrophoresis at 35 V for 15 minutes.

FIG. 19 shows the results of confirming mRNA binding for the mRNA-UIO66MN complex at varying concentrations. It was confirmed that no mRNA was detected, unlike the mRNA control, indicating that the mRNA in the mRNA-UIO66MN complex was well bound.

FIG. 20 shows the results of confirming mRNA binding for the mRNA-UIO66APD complex at varying concentrations. It was confirmed that no mRNA was detected, unlike the mRNA control, indicating that the mRNA in the mRNA-UIO66APD complex was well bound.

Although the preferred embodiments of the present invention have been described in detail above, the scope of the present invention is not limited thereto, and various modifications and improvements can be made by those skilled in the art without departing from the basic concept of the present invention as defined in the following claims and also fall within the scope of rights of the present invention.

This project (result) is the result of a local government-university cooperation-based regional innovation project (2021RIS-001) supported by the National Research Foundation of Korea funded by the Ministry of Education in 2024.
[Project Serial Number] 1345370811
[Project Number] 2021RIS-001
[Government Department] The Ministry of Education (P13)
[Project Management Agency] The National Research Foundation of Korea
[Research Project Name] Local government-university cooperation-based regional innovation project
[Research Task Name] Local government-university cooperation-based regional innovation project (Chungbuk regional innovation platform)
[Contribution Rate]
[Agency Carrying Out Project] (Chungbuk regional innovation platform) Chungbuk National University
[Research Period] March 1, 2024 to February 28, 2025

### Industrial Applicability

The present invention relates to a novel metal-organic framework and a method for producing the same.

## Claims

1. A novel metal-organic framework surface-modified with a compound represented by Formula 1 below: 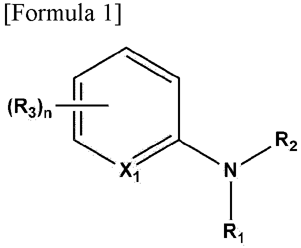 wherein
n is an integer ranging from 0 to 4,
X₁ is C(R₄) or N, and
R₁ to R₄ are the same as or different from one another and are each independently selected from the group consisting of hydrogen, a cyano group, a nitro group, a halogen group, a hydroxy group, a substituted or unsubstituted alkylthio group having 1 to 4 carbon atoms, a substituted or unsubstituted alkyl group having 1 to 30 carbon atoms, a substituted or unsubstituted cycloalkyl group having 3 to 20 carbon atoms, a substituted or unsubstituted alkenyl group having 2 to 30 carbon atoms, a substituted or unsubstituted alkynyl group having 2 to 24 carbon atoms, a substituted or unsubstituted aralkyl group having 7 to 30 carbon atoms, a substituted or unsubstituted aryl group having 6 to 30 carbon atoms, a substituted or unsubstituted heteroaryl group having 1 to 60 carbon atoms, a substituted or unsubstituted heteroarylalkyl group having 2 to 30 carbon atoms, a substituted or unsubstituted alkoxy group having 1 to 30 carbon atoms, a substituted or unsubstituted alkylamino group having 1 to 30 carbon atoms, a substituted or unsubstituted arylamino group having 6 to 30 carbon atoms, a substituted or unsubstituted aralkylamino group having 7 to 30 carbon atoms, a substituted or unsubstituted heteroarylamino group having 1 to 24 carbon atoms, a substituted or unsubstituted alkylsilyl group having 1 to 30 carbon atoms, a substituted or unsubstituted arylsilyl group having 6 to 30 carbon atoms, and a substituted or unsubstituted aryloxy group having 6 to 30 carbon atoms.

2. The novel metal-organic framework of claim 1, wherein the compound represented by Formula 1 is a compound represented by Formula 2 below: 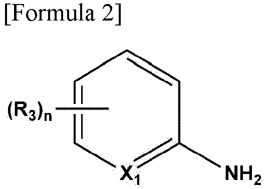 wherein
n, X₁ and R₃ are as defined in claim 1.

3. The novel metal-organic framework of claim 1, wherein the metal-organic framework comprises a metal selected from the group consisting of Li, Na, Mg, Ca, Sr, Ba, Sc, Y, Ti, Zr, Hf, V, Nb, Ta, Cr, Mo, W, Mn, Re, Fe, Ru, Os, Co, Rh, Ir, Ni, Pd, Pt, Cu, Ag, Au, Zn, Cd, Hg, Al, Ga, In, Tl, Si, Ge, Sn, Pb, As, Sb and Bi, or a metal ion selected from the group consisting of Li⁺, Na⁺, Mg²⁺, Ca²⁺, Sr²⁺, Ba²⁺ , Sc³⁺, Y³⁺, Ti⁴⁺, Zr⁴⁺, Hf⁴⁺, V⁴⁺, V³⁺, V²⁺, Nb³⁺, Ta³⁺, Cr³⁺, Mo³⁺, W³⁺, Mn³⁺, Mn²⁺ , Re³⁺, Re²⁺, Fe³⁺, Fe²⁺, Ru³⁺, Ru²⁺, Os³⁺, Os²⁺, Co³⁺, Co²⁺, Rh²⁺, Rh⁺, Ir³⁺, Ir²⁺, Ir⁺, Ni²⁺, Ni⁺, Pd²⁺, Pd⁺, Pt²⁺, Pt⁺, Cu²⁺, Cu⁺, Ag⁺, Au⁺, Zn²⁺, Cd²⁺, Hg²⁺, Al³⁺, Ga³⁺, In³⁺, Tl³⁺, Si⁴⁺, Si²⁺, Ge⁴⁺, Ge²⁺, Sn⁴⁺, Sn²⁺, Pb⁴⁺, Pb²⁺, As⁵⁺, As³⁺, As⁺, Sb⁵⁺, Sb³⁺, Sb⁺, Bi⁵⁺, Bi³⁺ and Bi⁺.

4. The novel metal-organic framework of claim 1, wherein the metal-organic framework is selected from the group consisting of an aluminum-based metal-organic framework, an iron-based metal-organic framework, a zirconium-based metal-organic framework, and mixtures thereof.

5. The novel metal-organic framework of claim 1, wherein the metal-organic framework is selected from the group consisting of UIO-66, UIO-66-NH₂, UIO-67, UIO-67-NH₂, PCN-128, PCN-222, PCN-223, PCN-224, MOF-525, MOF-545, MOF-801, MOF-808, MOF-867, Al-MIL-53, Al-MIL-53-NH₂, Al-MIL-88, Al-MIL-88-NH₂, Al-MIL-100, Al-MIL-100-NH₂, Al-MIL-101, Al-MIL-101-NH₂, Al-MIL-125, Al-MIL-125-NH₂, Fe-MIL-53, Fe-MIL-53-NH₂, Fe-MIL-88, Fe-MIL-88-NH₂, Fe-MIL-100, Fe-MIL-100-NH₂, Fe-MIL-101, Fe-MIL-101-NH₂, Fe-MIL-125, and Fe-MIL-125-NH₂.

6. The novel metal-organic framework of claim 1, wherein the metal-organic framework has a specific surface area of 1,400 m²/g to 1,500 m²/g.

7. The novel metal-organic framework of claim 1, wherein the metal-organic framework has a pore diameter of 1 nm to 3 nm.

8. A method for producing a novel metal-organic framework, comprising steps of:
preparing a metal-organic framework-containing solution by suspending a metal-organic framework in a first organic solvent;
preparing a surface-modifying solution by dissolving a compound represented by Formula 1 below in a second organic solvent; and
mixing and stirring the metal-organic framework-containing solution and the surface-modifying solution: 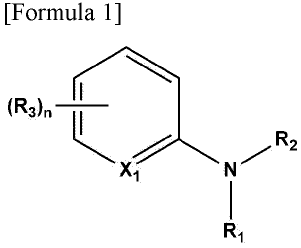
wherein
n is an integer ranging from 0 to 4,
X₁ is C(R₄) or N, and
R₁ to R₄ are the same as or different from one another and are each independently selected from the group consisting of hydrogen, a cyano group, a nitro group, a halogen group, a hydroxy group, a substituted or unsubstituted alkylthio group having 1 to 4 carbon atoms, a substituted or unsubstituted alkyl group having 1 to 30 carbon atoms, a substituted or unsubstituted cycloalkyl group having 3 to 20 carbon atoms, a substituted or unsubstituted alkenyl group having 2 to 30 carbon atoms, a substituted or unsubstituted alkynyl group having 2 to 24 carbon atoms, a substituted or unsubstituted aralkyl group having 7 to 30 carbon atoms, a substituted or unsubstituted aryl group having 6 to 30 carbon atoms, a substituted or unsubstituted heteroaryl group having 1 to 60 carbon atoms, a substituted or unsubstituted heteroarylalkyl group having 2 to 30 carbon atoms, a substituted or unsubstituted alkoxy group having 1 to 30 carbon atoms, a substituted or unsubstituted alkylamino group having 1 to 30 carbon atoms, a substituted or unsubstituted arylamino group having 6 to 30 carbon atoms, a substituted or unsubstituted aralkylamino group having 7 to 30 carbon atoms, a substituted or unsubstituted heteroarylamino group having 1 to 24 carbon atoms, a substituted or unsubstituted alkylsilyl group having 1 to 30 carbon atoms, a substituted or unsubstituted arylsilyl group having 6 to 30 carbon atoms, and a substituted or unsubstituted aryloxy group having 6 to 30 carbon atoms.

9. The method of claim 8, wherein the step of stirring is performed by stirring at 80°C to 120°C for 4 to 8 hours to surface-modify the metal-organic framework.

10. The method of claim 8, wherein the step of stirring is followed by washing and drying steps, thereby producing a surface-modified metal-organic framework.

11. The method of claim 10, wherein the washing step comprises steps of:
subjecting a reaction product resulting from the step of stirring to a first centrifugation at 5,000 rpm to 15,000 rpm for 1 to 10 minutes; suspending a precipitate, separated by the first centrifugation, in a third organic solvent, and then subjecting the suspension to a first washing by repeating a second centrifugation at 5,000 rpm to 15,000 rpm for 1 to 10 minutes 1 to 5 times; and
suspending a precipitate, separated by the first washing, in a fourth organic solvent, and then subjecting the suspension to a second washing by repeating a second centrifugation at 5,000 rpm to 15,000 rpm for 1 to 10 minutes 1 to 5 times.

12. The method of claim 10, wherein the drying step is performed by drying lower-layer particles, separated by the second washing, in an oven at 60°C to 100°C for 2 to 6 hours.
